## Europäisches Patentamt
## European Patent Office
## Office européen des brevets

(19)

(11) Veröffentlichungsnummer: **0 130 315**
**B1**

(12) # EUROPÄISCHE PATENTSCHRIFT

(45) Veröffentlichungstag der Patentschrift:
**12.10.88**

(21) Anmeldenummer: **84105228.5**

(22) Anmeldetag: **09.05.84**

(51) Int. Cl.⁴: **A 01 N 31/02**, A 61 L 2/00,
A 61 L 2/18

(54) **Reinigungsbehandlung von Blutkompartments von Dialysatoren.**

(30) Priorität: **28.05.83 DE 3319504**

(43) Veröffentlichungstag der Anmeldung:
**09.01.85 Patentblatt 85/2**

(45) Bekanntmachung des Hinweises auf die Patenterteilung:
**12.10.88 Patentblatt 88/41**

(84) Benannte Vertragsstaaten:
**BE DE FR GB IT SE**

(56) Entgegenhaltungen:
**DE-A-2 456 174**
**DE-C-2 749 516**

**CHEMICAL ABSTRACTS, Band 89, Nr. 2, 10. Juli 1978, Seite 376, Nr. 12200r, Columbus, Ohio, US; & JP - A - 77 155 219 (NIPPON ZEON CO., LTD.) 23.12.1977**
**CHEMICAL ABSTRACTS, Band 88, Nr. 16, 17. April 1978, Seite 335, Nr. 110553g, Columbus, Ohio, US; & JP - A - 77 125 475 (NIPPON ZEON CO., LTD.) 21.10.1977**
**CHEMICAL ABSTRACTS, Band 92, Nr. 16, 21. April 1980, Seite 404, Nr. 135140r, Columbus, Ohio, US; & JP - A - 79 132 241 (DAISAN GOMAI K.K.) 15.10.1979**

(73) Patentinhaber: **Akzo Patente GmbH, Postfach 10 01 49 Kasinostrasse 19- 23, D-5600 Wuppertal- 1 (DE)**

(72) Erfinder: **Henne, Werner, Dr., Winterbergstrasse 46, D-5600 Wuppertal 2 (DE)**
Erfinder: **Pelger, Michael, Dr., Buschenburg 32, D-5600 Wuppertal 22 (DE)**

## Beschreibung

In der Hämodialyse haben sich Hohlfaserdialysatoren speziell aus Cellulose weltweit gegenüber solchen aus Flach- und Schlauchmembranen durchgesetzt, weil sie offenbar eine Reihe von Vorteilen nicht nur in der raumsparenden Bauweise, der hohen Effizienz und der leichten Handhabung, sondern auch im Verhalten zum Humanblut durch gut geordnete Strömungsaufteilung besitzen.

Cellulosische Hohlfasern wie z. B. solche, die nach dem Cuprammonium-Verfahren hergestellt werden, leiden aber unter dem Nachteil, daß ihre Innenseite (= spätere Blutseite) im Herstellungsprozeß nicht dem gleichen intensiven Waschprozeß ausgesetzt, sondern praktisch nur von der Außenseite her gewaschen werden kann. Diese Tatsache führt zu einem Unterschied im Restgehalt an extrahierbaren noch unbekannten Substanzen im Molgewichtsbereich zwischen ca. 1 000 und 100 000.

Es ist gegenwärtig noch unbekannt, welche chemische Zusammensetzung diese Substanzen besitzen. Vermutungen wurden aber geäußert, daß sie evtl. für gelegentlich auftretende anaphylaktische oder andere Unverträglichkeitsreaktionen bei Dialysepatienten entweder allein oder in Verbindung mit Äthylenoxyd (Sterilisationsmittel) verantwortlich sind.

Aus der DE-OS-2 811 551 war es bekannt, einer Heißwassersterilisation eine Waschstufe vorzuschalten und die Sterilisation an einem mit Wasser oder physiologischer Kochsalzlösung gefüllten Dialysator durchzuführen, d. h. mit Wasser oder im nassen Zustand an die Klinik ausgeliefert.

Aus der JP-OS-52-155 219 ist ein Verfahren zur Behandlung von Hohlfasern, wie sie in Hämodialysatoren Verwendung finden, bekannt. Bei dieser Behandlung werden in Wasser schwerlösliche, organische Substanzen mit Methanol, das 0 bis 10 Gew.-% Wasser enthält, gewaschen und anschließend mit Methanol, Ethanol, n-Propanol oder Isopropanol von denen mindestens eine Art ausgewählt wird, behandelt, wobei dieses Behandlungsmittel mindestens 0,5 Gew.-% Glyzerin und 0 bis 10 Gew.-% Wasser enthält. Anschließend werden die Hohlfäden getrocknet.

Es war nun die Aufgabe gestellt, ein Verfahren zur Reinigung von Hohlfaser-Dialysatoren in der Fertigungsstufe der Dialysatoren zu finden, das den Nachteil der fehlenden Wasserwasche auf der Hohlfaserinnenseite aufhebt und eine Möglichkeit schafft, im Bedarfsfall ohne Äthylenoxyd-Sterilisation auszukommen. (Das Hohlfaserinnere ist von der Produktion her mit einer Füllflüssigkeit gefüllt.)

Bisher bekannte Wege hierzu sind die Herstellung wassergefüllter Hohlfaser-Dialysatoren in Verbindung mit Formalinzusatz oder in Verbindung mit Heißdampfsterilisation oder die γ-Sterilisation. In allen Fällen wird es vom Dialysatorhersteller als sehr lästig und unökonomisch empfunden, daß mit jedem Dialysator ein hohes Flüssigkeitsgewicht von einigen zusätzlichen hundert Gramm zu verschicken ist, während die Kliniken (Verbraucher der Dialysatoren) die Unhandlichkeit und den langen Zeitbedarf zum Auswaschen vor Behandlungsbeginn beanstanden.

Trotz der oben geschilderten Nachteile werden von den Anwendern "trocken" gelieferte Dialysatoren klar bevorzugt, jedoch haben sich z. B. trockene, γ-bestrahlte Dialysatoren nicht durchsetzen lassen, weil die Bestrahlung die hydraulische Permeabilität von Cellulose-Hohlfasern um rund 40 % erniedrigt. Die Wasserwäsche mit anschließender Trocknung hat sich am fertigen Dialysator aber nicht durchführen lassen, weil die Hohlfasern bei der Trocknung so stark schrumpfen (um ca. 8 %), daß die resultierende Schrumpfkraft entweder zur Zerstörung der Kopfenden, zum Einziehen der Einbettungsschicht oder zum Herauslösen einzelner Fasern und zu Undichtigkeiten führt.

Zur Lösung der geschilderten Aufgabe wurde das folgende erfolgreiche Verfahren gefunden:

Man wäscht das Blutkompartment des fertig montierten Dialysators, dessen Innenflüssigkeit vorher auf üblichem Weg entfernt worden war, gründlich mit reinem Wasser oder mit Wasser, das Elektrolyte und/oder organische Lösungsmittel enthält, in einem geschlossenen Kreislauf auf der Blutseite (Blutkompartment) etwa innerhalb 5 bis 15 Minuten, entleert und füllt das Blutkompartment des Dialysators anschließend mit einer ternären Lösung aus Wasser + Glycerin + Alkohol die mindestens 25 % Glycerin enthält, entleert nach kurzer Zeit und trocknet. Dabei stellt man ab einem Glyceringehalt des Gemisches ab 25 % (Gew.-%) überraschend fest, daß die Fasern beim Trocknen nicht schrumpfen und die Ultrafiltrationsrate nicht abfällt, während sie sonst bei der Trocknung eines nassen Fadens stets abfällt.

Der trockene Hohlfaser-Dialysator wird dann sterilisiert, was mit Äthylenoxyd und/oder mit γ-Strahlen und/oder Heißdampf geschehen kann.

Die erfindungsgemäß behandelten sterilisierten Dialysatoren können, soweit nicht eine Äthylenoxydsterilisation vorgenommen wurde, in diesem Zustand sofort ausgeliefert werden. (Im Falle der Äthylenoxyd-Sterilisation ist üblicherweise eine Quarantänezeit zur Ausgasung von etwa vier Wochen erforderlich.)

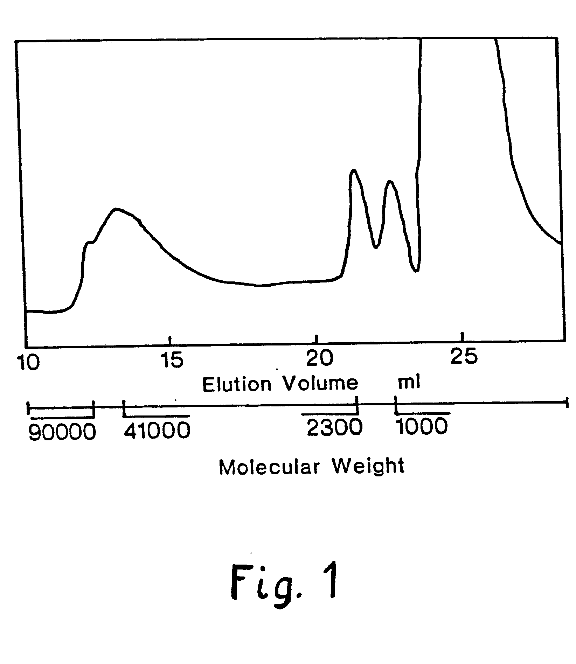

Das Chromatogramm (Figur 1) wurde an blutseitig erhaltenen Extrakten aus Hohlfaser-Dialysatoren gewonnen, wie sie üblicherweise im Verkehr sind, gleichgültig, ob dieselben im Fertigungsprozeß mit hydrophoben Lösungsmitteln vom Freontyp (Trichlortrifluoräthan 113), mit Alkoholen wie Äthanol, Methanol, Isopropanol oder mit Gemischen derselben gereinigt worden waren. Die Grafik zeigt Peaks für Substanzen im Bereich von 90 000, 40 000, 2 300 und 1 000 auf. Diese Molekulargewichte wurden den Substanzen nach einer Eichung mit Pullulanen zugeordnet.

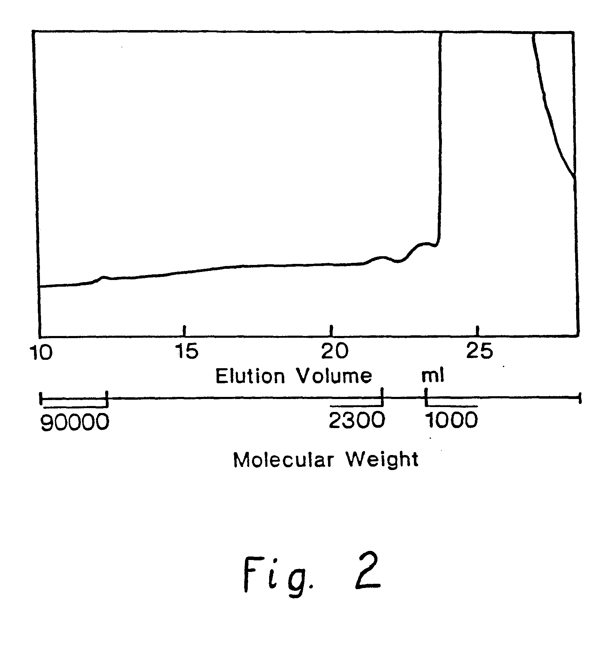

Werden Dialysatoren am Ende des Dialysatorfertigungsprozesses dem geschilderten erfindungsgemäßen Verfahren unterzogen und später blutseitig (auf der Innenseite) mit Wasser extrahiert, dann ist dieser Extrakt praktisch frei von den Peaks (s. Figur 2).

Die Figur 2 gilt auch für einen Cellulose-Hohlfaser-Dialysator, der bei der Auslieferung in die Klinik noch extrahierbare Anteile besaß, in der Klinik jedoch außergewöhnlich lange mit Wasser gespült und gründlich mittels Wasserdurchlauf im single pass endgereinigt wurde. Deshalb ist es von großem Vorteil, wenn die Dialysatoren bereits den Fertigungsprozeß mit der geschilderten Freiheit von durch Wasser extrahierbaren Substanzen verlassen.

Bei den Versuchen zu obigem Problem wurde ein weiterer bisher unbekannter Effekt gefunden: Wird in der ternären Lösung der Glyceringehalt über 50 % (Gew.-%) gesteigert bei gleicher oder unterschiedlicher Aufteilung des Restes unter die beiden anderen Komponenten Alkohol + Wasser, dann wird die Ultrafiltrationsrate des Dialysators gegenüber dem Ausgangswert sogar noch gesteigert. Man kann also trotz nachträglicher Trocknung bzw. $\gamma$-Sterilisation mit der Höhe des Glycerinzusatzes die Ultrafiltrationsrate des Dialysators steigern und somit das erfindungsgemäße Verfahren in zusätzlicher Weise vorteilhaft nutzen.

### Beispiel 1

Ein Cellulose-Hohlfaserdialysator des Typs CF 1211, der Firma Baxter Travenol Inc., wurde 15 Minuten lang blutseitig im Kreislauf bei einem Fluß von 200 ml/min mit Reinstwasser von Raumtemperatur gespült. Das Gesamtvolumen des Wassers betrug 2 l. Nach Ablassen des Wassers wurde der Dialysator mit einer Lösung aus

50 % Isopropanol
25 % Glycerin
25 % Wasser

blutseitig bei Raumtemperatur gefüllt, 5 Minuten stehengelassen, anschließend drainiert und 3 Stunden bei 50° C im Vakuum getrocknet. Der Dialysator blieb dicht, die Fasern blieben einzeln und unverklebt liegen, die kopfseitigen Einbettschichten blieben unverletzt.

Anschließend wurde die Ultrafiltrationsrate mit Wasser geprüft, sie betrug 4,3 ml/h . m2 . mm Hg, was dem Ausgangswert der verwandten Fasertype (Cellulose-Hohlfaser nach dem Cuprammoniumverfahren hergestellt) entsprach. Ein befürchteter 40 %-iger Abfall war vermieden.

Anschließend erfolgten Extraktion und Analyse analog Vergleichsbeispiel 1. Das erhaltene HLP-Chromatogramm ist in Figur 2 dargestellt.

### Beispiel 2

Ein zweiter Dialysator des gleichen Lots, der in gleicher Weise mit Wasser gespült wurde, dann aber an Stelle der obigen Lösung nur mit Wasser gefüllt und in gleicher Weise wie oben weiterbehandelt wurde, überstand die Trocknung nicht. Die PU-Einbettschicht wurde 2 mm weit nach innen gezogen und wurde undicht, so daß keine Messung der Ultrafiltrationsrate mehr möglich war.

Das erfindungsgemäße Verfahren eignet sich gleichermaßen zur Behandlung von Flachmembran- und Schlauchmembrandialysatoren, wobei der Steigerung der Filtrationsrate besondere Bedeutung zukommt.

### Patentansprüche

1. Verfahren zur Reinigungsbehandlung des Blutkompartments von fertig montierten Dialysatoren, die Membranen aus Zellulose in Form von Hohlfäden, Schläuchen oder Flachfolien enthalten noch in der Fertigungsstufe, gekennzeichnet durch folgende Maßnahmen:

a) Waschen des Blutkompartments mit reinem Wasser oder mit Wasser, das Elektrolyte und/oder organische Lösungsmittel enthält, in einem geschlossenen Kreislauf und anschließendem Entleeren und dann

b) Auffüllen mit einer ternären Lösung aus Wasser, Glycerin und Alkohol, wobei die Lösung mindestens 25 % Glyzerin enthält, und nach kurzer Zeit Entleeren und Trocknen

c) Sterilisieren mit $\gamma$-Strahlen und/oder Äthylenoxyd und/oder Heißdampf.

2. Verfahren nach Anspruch 1, dadurch gekennzeichnet, daß die Membranen aus Cellulose-Cuoxam-Lösungen hergestellt wurden.

### Revendications

1. Procédé pour nettoyer le compartiment côté sang de dialyseurs entièrement montés, contenant des membranes en cellulose sous forme de fibres creuses, de tubes souples ou de feuilles planes, encore au stade de fabrication, caractérisé en ce qu'il comprend les stades opératoires suivants:

a) on lave le compartiment côté sang avec de l'eau pure ou de l'eau contenant des électrolytes et/ou des solvants organiques, en circuit fermé, puis on vidange, puis

b) on remplit d'une solution ternaire d'eau, de glycérol et d'alcool, cette solution contenant au moins 25 % de glycérol et, après un court moment, on vidange et on sèche,
   c) on stérilise aux rayons gamma et/ou à l'oxyde d'éthylène et/ou à la vapeur surchauffée.
   2. Procédé selon la revendication 1, caractérisé en ce que les membranes ont été préparées à partir de solutions de cellulose-cuoxam.


## Claims

1. Process for the cleansing treatment of the blood compartment of mounted dialysers containing membranes of cellulose in the form of hollow fibres, tubes or flat films while still in the manufacturing stage, characterised by the following measures:
   a) washing of the blood compartment with pure water or with water containing electrolytes and/or organic solvents in a closed circulation followed by emptying and then
   b) filling up with a ternary solution of water, glycerol and alcohol, which solution contains at least 25 % of glycerol, and then emptying and drying after a short time, and
   c) sterilization with γ-rays and/or ethylene oxide and/or superheated steam.
   2. Process according to claim 1, characterised in that the membranes were produced from cellulose-cuoxam solutions.

Fig. 1

Fig. 2